(19) **Europäisches Patentamt / European Patent Office / Office européen des brevets**

(11) **EP 2 995 685 A1**

(12) # EUROPEAN PATENT APPLICATION
published in accordance with Art. 153(4) EPC

(43) Date of publication:
**16.03.2016 Bulletin 2016/11**

(21) Application number: **14794926.7**

(22) Date of filing: **08.05.2014**

(51) Int Cl.:
*C12P 19/14* [(2006.01)]     *B09B 3/00* [(2006.01)]
*C02F 11/02* [(2006.01)]     *C13K 1/02* [(2006.01)]

(86) International application number:
**PCT/JP2014/062328**

(87) International publication number:
**WO 2014/181818 (13.11.2014 Gazette 2014/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.05.2013   JP 2013098339**
**17.07.2013   JP 2013148544**
**12.11.2013   JP 2013234005**
**28.02.2014   JP 2014038355**

(71) Applicants:
• **Acteiive Corporation**
**Chiba 278-8510 (JP)**
• **Abe, Masahiko**
**Noda-city, Chiba 278-0017 (JP)**
• **Sakaguchi, Kengo**
**Tsukuba-shi, Ibaraki 300-2667 (JP)**
• **Kido, Shigeru**
**Ishikawa-gun, Fukushima 963-7808 (JP)**

(72) Inventors:
• **ABURAI Kenichi**
**Kasukabe-shi**
**Saitama 344-0056 (JP)**
• **KIKUCHI Yukiko**
**Sapporo-shi**
**Hokkaido 005-0015 (JP)**
• **YOSHIMOTO Ryo**
**Chigasaki-shi**
**Kanagawa 253-0088 (JP)**
• **KANAI Yoshihiro**
**Takasaki-shi**
**Gunma 370-0076 (JP)**
• **SEKI Yasutaka**
**Koshigaya-shi**
**Saitama 343-0025 (JP)**

(74) Representative: **Grünecker Patent- und Rechtsanwälte**
**PartG mbB**
**Leopoldstraße 4**
**80802 München (DE)**

(54) ## GLUCOSE PRODUCTION METHOD AND GLUCOSE PRODUCED BY SAID METHOD

(57)     An object of the present invention is to actualize a glucose production method for producing glucose from excrement X from living matter that is disposed as waste, without consuming food resources or agricultural resources, and to stably provide glucose at low cost without being affected by crop yields.

    The invention is a glucose production method characterized in that glucose is produced from cellulose contained in excrement X from living matter, and glucose produced by this method.

Fig. 1

EXCREMENT

S1 — STERILIZING STEP → Xs

S2 — WASHING STEP → C

S3 — PULVERIZING STEP → Cf

S4 — ENZYMOLYSIS STEP

S5 — GLUCOSE PURIFYING STEP

GLUCOSE

EP 2 995 685 A1

**Description**

TECHNICAL FIELD

The present invention relates to a glucose production method for producing glucose from excrement from living matter and glucose produced by this method.

BACKGROUND ART

In recent years, there has been an increase in the use of glucose in industrial applications as a starting material for bioethanol and polymeric materials that serve as alternative fuels for petroleum fuel.
Conventionally, as described in Patent Literature 1, glucose for industrial use has been produced from potatoes, grains such as corn, wheat, barley, rye, triticale, and rice, or plants used as raw material for sugar such as sugarcane and sugar beets.
In addition to the foregoing, Patent Literature 2 describes producing glucose from cellulose found in waste timber and thinnings, which include conifers such as cedar and cypress, and broadleaved trees such as beech and oak, and wood-based waste such as waste paper.
Patent Literature 1: Japanese Patent Laid-open Publication No. 2011-519550
Patent Literature 2: Japanese Patent Laid-open Publication No. 2006-149343

DISCLOSURE OF INVENTION

PROBLEM TO BE SOLVED BY THE INVENTION

However, while sugar resources, such as grains and plants used as raw material for sugar, are basically produced as food resources, should industrial application of sugar resources expand in the future, sugar resources are likely to be consumed as industrial resource. If the consumption of sugar resources as industrial resource increases, there is concern that the market prices of sugar resources as food will rise, thereby cutting into household budgets and accelerating famine in developing countries.
In addition, waste timber, thinnings, and wood-based waste such as waste paper are useful resources that can be recycled into disposable chopsticks, toothpicks, newspapers, toilet paper, biodegradable plastics, and the like. Waste timber and thinnings can also be used as agricultural resources. Furthermore, cellulose in wood-based waste and the like contain components that inhibit decomposition. Therefore, there is a problem in that such inhibitory components make glycosylation of cellulose difficult.
Here, in Japan, about 40 million tons of bovine feces and about 13 million tons of bovine urine are expelled annually from dairy cattle and beef cattle bred as livestock. Most of the bovine feces and bovine urine are deposited, or undergo simple composting, and are then disposed.
The inventors of the present invention and the like have focused on the use of cellulose contained in excrement from livestock, such as dairy cattle and beef cattle, and more broadly, from living matter, as a cellulose raw material for which a stable supply is obtainable. As a result of keen research into methods for producing glucose from excrement, the inventors of the present invention and the like have completed the present invention.
Here, an object of the present invention is to actualize a glucose production method for producing glucose from excrement from living matter that is disposed as waste, without consuming food resources or agricultural resources, and stably provide glucose at low cost without being affected by crop yields.

MEANS FOR SOLVING PROBLEM

To achieve the above-described object, a first aspect of the glucose production method of the present invention is characterized in that glucose is produced from excrement from living matter. In addition, a second aspect of the glucose production method of the present invention is characterized in that glucose is produced from cellulose contained in the excrement. Furthermore, a third aspect of the glucose production method of the present invention is characterized in that a decomposing step of decomposing the cellulose contained in the excrement to glucose using a cellulolytic enzyme is performed.
According to the first aspect to third aspect of the glucose production method of the present invention such as that described above, through use of excrement from living matter, which is ordinarily a waste product, glucose can be manufactured without consuming food resources such as starches or recyclable resources such as wood-based waste.

A fourth aspect of the glucose production method of the present invention is characterized in that a pulverizing step of pulverizing the cellulose contained in the excrement is performed before the decomposing step. In addition, a fifth aspect of the glucose production method of the present invention is characterized in that, at the pulverizing step, the cellulose contained in the excrement is pulverized into fine particles having a diameter of 40 μm or less.

According to the fourth aspect and the fifth aspect of the glucose production method of the present invention, as a result of the pulverizing step being performed, amorphous regions in the cellulose are exposed, and cellulose of which enzymolysis can be easily performed by a cellulolytic enzyme is obtained. Therefore, glucose can be produced at a high yield from the cellulose contained in the excrement.

The glucose of the present invention is characterized by being produced from excrement from living matter by a glucose production method according to any of the first aspect to fifth aspect.

In the glucose of the present invention such as that above, through use of a raw material, that is, excrement from living matter of which a very stable and low-cost supply can be expected, glucose can be stably produced at low cost without being affected by the status of crop yields and the like.

EFFECT OF THE INVENTION

In the glucose production method of the present invention, a glucose production method can be provided for producing glucose from excrement from living matter that is disposed as waste, without consuming food resources or agricultural resources.

In addition, in the glucose of the present invention, glucose that can be stably produced without being affected by the status of crop yields can be provided.

BRIEF DESCRIPTION OF DRAWINGS

Fig. 1 is a flowchart of production steps in a glucose production method according to an embodiment of the present invention.

Fig. 2 shows optical microscope observation images of raw materials during the production steps in an example of the glucose production method of the present invention, in which (a) shows sterilized bovine feces Ds, (b) shows cellulose raw material C, and (c) shows fine cellulose raw material Cf.

Fig. 3 is a graph of glucose yield from each sample in example 1 of the glucose production method of the present invention.

Fig. 4 is a graph of the correlation between the size of granularity at a pulverizing step S3 performed on each sample and glucose yield in example 2 of the glucose production method of the present invention.

Fig. 5 is a graph of powder X-ray analysis results indicating the correlation between the size of granularity at the pulverizing step S3 performed on each sample and the crystalline form of cellulose in example 2 of the glucose production method of the present invention.

Fig. 6 is a graph of the glucose yield from each sample in example 3 of the glucose production method of the present invention.

Fig. 7 is a graph of the glucose yield from each sample in example 4 of the glucose production method of the present invention.

Fig. 8 is a graph of the glucose yield from each sample in example 5 of the glucose production method of the present invention.

Fig. 9 is a graph of the glucose yield from each sample in example 6 of the glucose production method of the present invention.

Fig. 10 is a graph of the total sugar content in bovine feces.

BEST MODE(S) FOR CARRYING OUT THE INVENTION

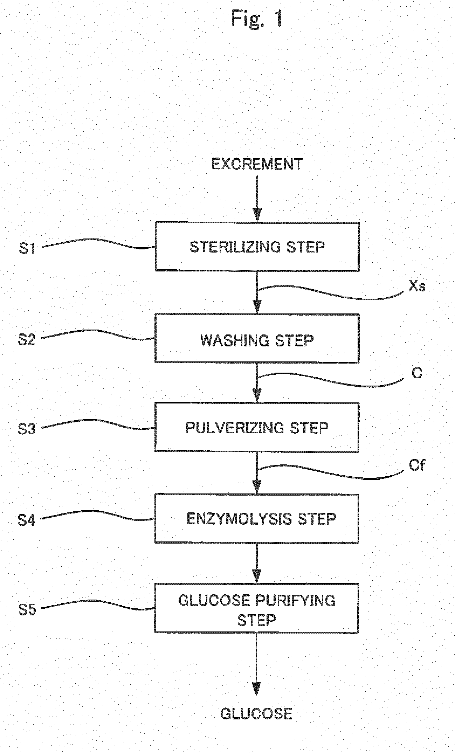

A specific embodiment of a glucose production method of the present invention will hereinafter be described in detail with reference to Fig. 1 to Fig. 3.

The glucose production method of the present invention is a method for producing glucose from excrement, such as feces and urine, from living matter.

The glucose production method of the present invention is a method for obtaining glucose, which is a monosaccharide, by decomposing cellulose, which is a polysaccharide, contained in excrement from living matter using a cellulolytic enzyme. Therefore, the living matter providing the excrement is preferably an herbivorous animal from which a large cellulose content can be expected. For example, livestock such as cattle, swine, poultry, rabbits, and horses, ornamental animals such as elephants, rhinoceros, giraffes, sheep, goats, deer, wild boars, guinea pigs, rats, and mice, insects such as silkworms and locusts, and marine life such as parrotfish, rabbitfish, and turbo cornutus are applicable as the living matter.

As long as the excrement from living matter is composed of urine as a liquid component and feces as a solid component and contains cellulose, either of the liquid component and the solid component, or a mixture thereof can be used as the excrement.

In addition, when the glucose production method of the present invention is used on an industrial production scale, in particular, through use of excrement provided by herbivorous animals bred as livestock, such as cattle, swine, poultry, or rabbits, or herbivorous animals raised for ornamental purposes in zoos and the like, such as elephants, rhinoceros, giraffes, sheep, or goats, a substantial amount of excrement can be stably and easily collected from a single location. As a result, glucose can be provided at low cost, while suppressing transport costs.

A cellulase containing three types of enzymes, endoglucanase, cellobiohydrolase, and β-glucosidase, can be used as the cellulolytic enzyme. Endoglucanase randomly cleaves cellulose at amorphous regions and thereby reduces the degree of polymerization of cellulose. Cellobiohydrolase decomposes cellulose in cellobiose units, from crystalline region terminals. β-glucosidase decomposes cellobiose into glucose.

The type of cellulase is not particularly limited, and may be a commercially available cellulase, or a cellulase derived from bacteria, plants, or the like. However, glucose yield can be improved through use of a cellulase derived from Trichoderma, which is excellent for cellulose decomposition.

According to the specific embodiment of the glucose production method of the present invention such as that described above, as shown in Fig. 1, the following steps are performed: a sterilizing step S1 of sterilizing recovered excrement X from living matter and obtaining sterilized excrement Xs; a washing step S2 of removing excess components from the sterilized excrement Xs and obtaining a cellulose raw material C; a pulverizing step S3 of pulverizing the obtained cellulose raw material C into a size suitable for enzymolysis and obtaining a fine cellulose raw material Cf; an enzymolysis step S4 of decomposing the fine cellulose raw material Cf using a cellulolytic enzyme and producing glucose; and a glucose purifying step S5 of purifying the produced glucose to a desired state.

At the pulverizing step S3, in particular, the obtained fine cellulose raw material Cf is preferably pulverized to a particle size having a diameter of 40 $\mu$m or less. As a result of pulverization into the fine cellulose raw material Cf in this way, the surface area of the cellulose, which serves as a substrate, is increased. The frequency of contact between the substrate and the cellulolytic enzyme is increased. It is thought that, as a result, the quantity of glucose that is produced will increase and the glucose yield will increase. In addition, as a result of amorphous regions being exposed in part of the cellulose which serves as a substrate, it is thought that enzymolysis by the cellulolytic enzyme will be facilitated, and the glucose yield will be improved.

The sterilizing step S1, the washing step S2, and the pulverizing step S3 can be selected and performed in combination as appropriate, based on the state of the recovered excrement X. Any of these steps can be omitted if unnecessary.

Furthermore, in addition to the above-described steps, for example, a fermenting step of fermenting the recovered excrement X to improve glucose yield, and a liquefying step of dissolving the excrement X in an ionic liquid, a basic solvent such as ethylenediamine, or the like to improve fluidity of the cellulose contained in the excrement X can be used.

In the glucose production method of the present invention such as that described above, glucose can be produced from the excrement X from living matter, which is a waste product, without consuming food resources such as starches and sugar raw materials, and recyclable resources such as wood-based waste. In addition, as a result of the excrement X being recovered stably and at low cost from at least herbivorous animals such as livestock and ornamental animals, glucose can be stably produced at low cost even when the glucose production method of the present invention is implemented on an industrial production scale. Furthermore, the glucose produced by the glucose production method of the present invention is produced from waste product. Therefore, glucose can be supplied to the market at low cost.

Specific examples of the glucose production method of the present invention will be described below.

<Example 1>

In the present example, glucose was produced using bovine feces as the excrement X from living matter. The sterilizing step S1, the washing step S2, the pulverizing step S3, and the enzymolysis step S4, shown in Fig. 1, were performed. Processes such as those below were performed at the respective steps.

First, at the sterilizing step S1, 200 g of bovine feces D was placed in a deep petri dish. Autoclave treatment was performed for 20 minutes at saturation water vapor pressure at 120°C. Sterilized bovine feces Ds was thereby obtained.

Then, at the washing step S2, 150 mL of ultra-pure water (> 18.2 M$\Omega$cm$^{-1}$) was added to 50 g of the sterilized bovine feces Ds. The resultant was mixed for 5 minutes with a mixer. The mixture was then centrifuged for 10 minutes at 27000 G at 4°C, in a centrifuge. The solid component was thereby separated from the sterilized bovine feces Ds. 150 mL of ultra-pure water was added to the solid component, and the mixing process and the centrifugation were performed again under the same conditions. The cellulose raw material C was thereby obtained from the sterilized bovine feces Ds.

At the pulverizing step D3, the cellulose raw material C was pulverized in a mortar, and the fine cellulose raw material Cf was obtained. In the present example, the cellulose raw material C is pulverized in a mortar. However, micronization may be performed using a publically known pulverization apparatus, and the fine cellulose raw material Cf of a size suitable for enzymolysis at the subsequent step may be obtained.

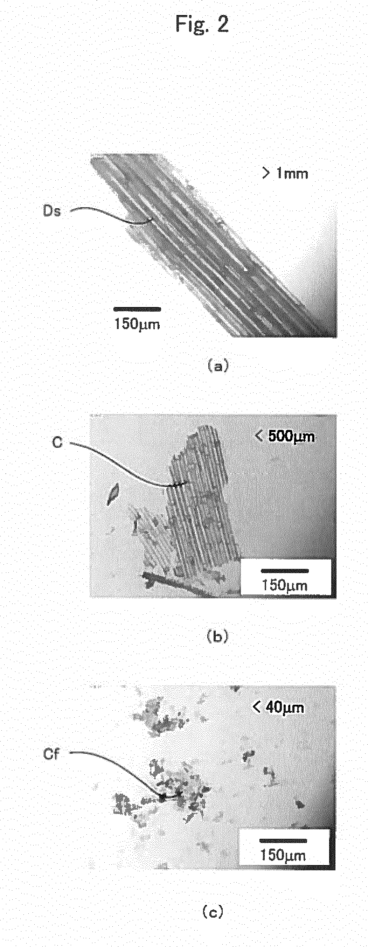

Here, the particle sizes of the sterilized bovine feces Ds obtained at the sterilizing step S1, the cellulose raw material C obtained at the washing step S2, and the fine cellulose raw material Cf obtained at the pulverizing step S3 are as follows. The sterilized bovine feces Ds is 1 mm or more as shown in Fig. 2(a). The cellulose raw material C is 500 $\mu$m or less as shown in Fig. 2(b). The fine cellulose raw material Cf is 40 $\mu$m or less as shown in Fig. 2(c). Fig. 2 shows the sterilized bovine feces Ds, the cellulose raw material C, and the fine cellulose raw material Cf observed under an optical microscope.

At the enzymolysis step S4, 10 mL of a 20 mM sodium acetate buffer solution (pH = 5.0) was added to 0.1 g of the fine cellulose raw material Cf, and a 1 wt% substrate dispersion was prepared. The 1 wt% substrate dispersion was divided among 2 mL microtubes in 200 $\mu$L units. The cellulolytic enzyme was a Trichoderma-derived cellulase. A cellulolytic enzyme solution comprising 500 $\mu$g/mL Trichoderma viride (manufactured by Sigma-Aldrich Corporation; code: C9422-10KU) / 20 mM sodium acetate buffer solution was used. 50 $\mu$L of the cellulolytic enzyme solution was added to 200 $\mu$L of the I wt% substrate dispersion, and 24-hour incubation was performed at 50°C.

In the present example, to compare the glucose yield from the bovine feces D and the glucose yield from hay H, which is cattle feed, the hay H was similarly subjected to the washing step S2, the pulverizing step S3, and the enzymolysis step S4, and glucose was collected therefrom. In addition, to examine the glucose yields based on differences in cellulose particle size, glucose was collected from the bovine feces D and the hay H by performing the enzymolysis step S4 on four types of samples: the cellulose raw material C and the hay H after the washing step S2, and the fine cellulose raw material Cf and fine hay Hf after the pulverizing step S3.

For quantitation of the glucose produced as a result of the enzymolysis step S4, 2 $\mu$L of supernatant was separated from each sample after incubation, and each supernatant was placed in a 1.5 mL tube. 300 mL of a glucose reagent (LabAssay (registered trademark) Glucose, manufactured by WAKO Pure Chemical Industries, Ltd.) was added to the supernatant, and the resultant was sufficiently fermented by incubation for 5 minutes at 37°C. 200 mL of the resultant was then poured onto a microplate (96 holes), and light absorbance at a wavelength of 505 nm was measured using a plate reader (SpectraMax (registered trademark) 190, manufactured by Molecular Devices, LLC.). The glucose quantity G (mg/g) obtained from 1 g of the sample upon drying was calculated from the slope k of the calibration curve using a glucose standard solution, using expression 1, below. Here, in expression 1, A represents the light absorbance of the sample, S represents the solution content of the sample, and M represents the weight of the sample upon drying.

[Expression 1]

$$G = \frac{\frac{A}{k} \times S}{M}$$

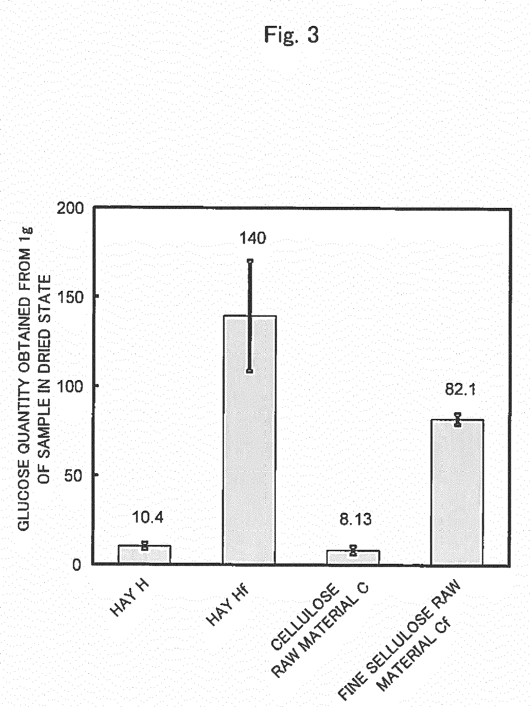

In the present example, as shown in Fig. 3, from 1 g of each sample upon drying: 8.13 mg of glucose was obtained from the cellulose raw material C; 82.1 mg of glucose was obtained from the fine cellulose raw material Cf; 10.4 mg of glucose was obtained from the hay H; and 140 mg of glucose was obtained from the fine hay H. The values indicated in the Fig. 3 are the mean values of three samples, and the standard deviations thereof are indicated as error bars.

In particular, it is confirmed that ten-times the glucose yield is obtained as a result of enzymolysis being performed using the fine cellulose raw material Cf and the fine hay H obtained at the pulverizing step S3, compared to the glucose yields when enzymolysis is performed on the cellulose raw material C and the hay H that have not undergone the pulverizing step S3.

From the above-described example, it is clear that glucose can be produced from the bovine feces D through use of the glucose production method of the present invention. Although the glucose yield is about 40% less than the glucose yield when the hay H is used, the bovine feces D can be stably supplied without being affected by the crop yield of hay H. Therefore, glucose can be stably produced.

<Example 2>

In the present example, the correlation between the size of granularity at the pulverizing step S3 of the bovine feces serving as the excrement X from living matter and the glucose yield, and the changes in cellulose crystalline form were determined.

In a manner similar to that in example 1, the sterilizing step S1, the washing step S2, and the pulverizing step S3 were performed on 1 g of the bovine feces serving as the excrement X from living matter. The obtained fine cellulose raw material Cf was separated into five types of particle size groups using sieves. The enzymolysis step S4 was performed on each particle size group, and glucose was thereby produced. The five types of particle size groups are, in order from the smallest to the largest in diameter: less than 53 $\mu$m; 53 $\mu$m or more and 90 $\mu$m or less; 90 $\mu$m or more and 150 $\mu$m or less; 150 $\mu$m or more and 300 $\mu$m or less; and 300 $\mu$m or more and 500 $\mu$m or less.

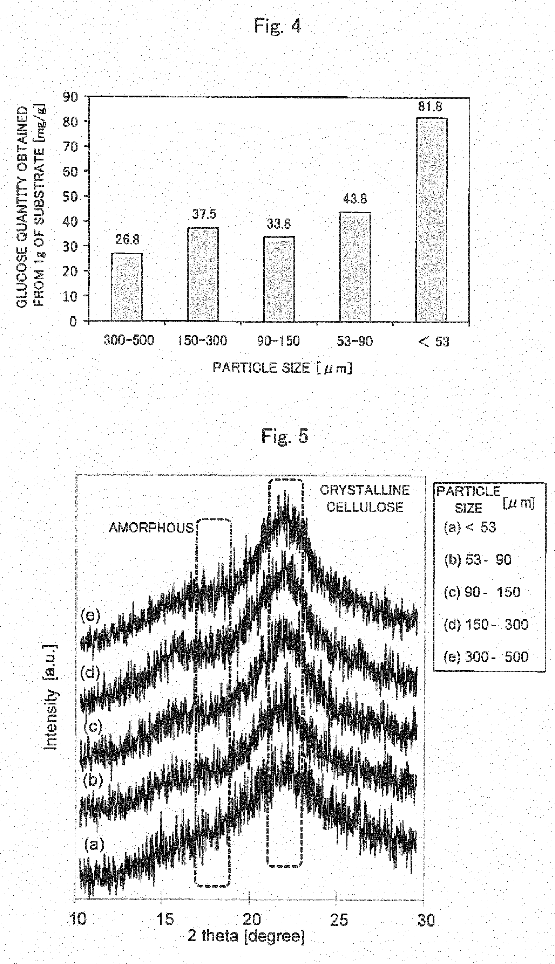

The mean glucose yield values after glucose has been produced three times in a similar manner are as shown in Fig. 4. Based on the results in Fig. 4, it is clear that the glucose yield increases in accompaniment with the decrease in particle size of the bovine feces serving as the excrement X from living matter, which serves as the substrate.

The changes in crystalline form were verified by measurement of the relationship between the angle of diffraction and intensity (arbitrary) using a power X-ray (by using analysis apparatus of PHILIPS X'Pert Pro (manufactured by Koninklijke Philips N.V.; CuK$\alpha$ beam: 0.154 nm)) on the produced cellulose. The results are as shown in Fig. 5. Based on the results in Fig. 5, it is clear that the crystalline form did not change in the majority portion of the cellulose as a result of mortar pulverization at the pulverization step S3.

<Example 3>

In the present example, bovine feces, swine feces, horse feces, chicken feces, and goat feces were used as the excrement X from living matter. The glucose yields thereof were determined.

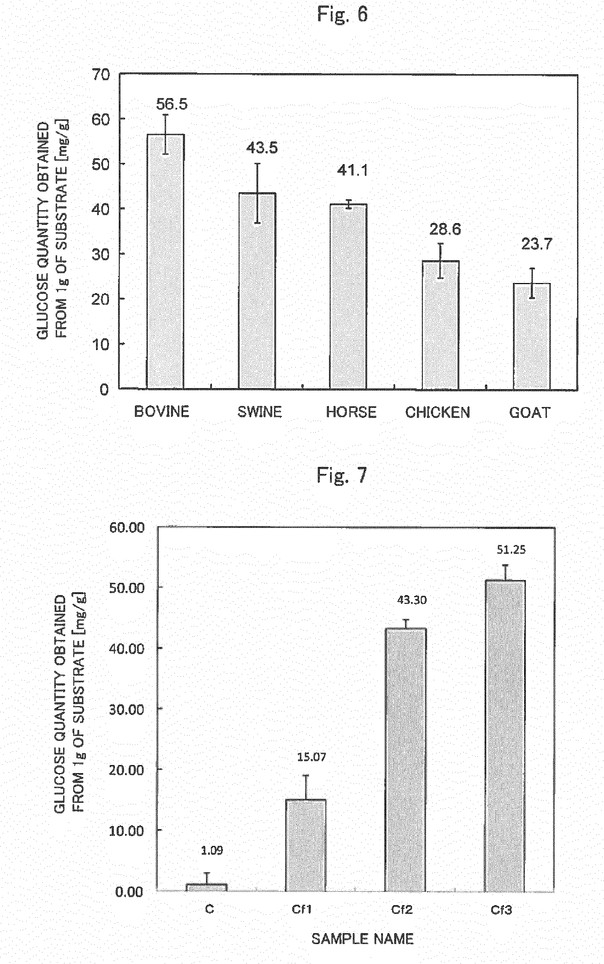

The mean glucose yield values after glucose has been produced three times under conditions similar to those in example 1 for each of the bovine feces, swine feces, horse feces, chicken feces, and goat feces are as shown in Fig. 6. Based on the results in Fig. 6, it is clear that glucose can be collected from the excrement from herbivorous animals other than bovine feces, as the excrement X from living matter serving as the substrate.

<Example 4>

In the present example, an instance in which a planetary ball mill is used at the pulverizing step S3 will be described. In the present example, glucose was produced by performing the sterilizing step S1, the washing step S2, the pulverizing step S3 using a mixer, a mortar, or a planetary ball mill, and the enzymolysis step S4 shown in Fig. 1. Processes such as those below were performed at the respective steps.

First, at the sterilizing step S1, 200 g of bovine feces D was placed in a deep petri dish. Autoclave treatment was performed for 20 minutes at saturation water vapor pressure at 120°C. Sterilized bovine feces Ds was thereby obtained.

At the washing step S2, 50 g of the sterilized bovine feces Ds was filtered and washed using a gauze and 3 L of ultra-pure water (> 18.2 M$\Omega$cm$^{-1}$). Then, the sterilized bovine feces Ds was dried for 16 hours at 50°C. 150 mL of ultra-pure water was added to the obtained dried matter. A mixing and pulverizing process was performed in a mixer at 15000 rpm for 10 minutes. The obtained pulverized matter was then separated into 50 mL units and centrifuged for 10 minutes at 27000 G at 4°C. The obtained precipitate was then dried for 16 hours at 50°C, and the cellulose raw material C was thereby obtained from the sterilized bovine feces Ds (sample C).

At the pulverizing step S3 using a mixer, 200 mL of ultra-pure water was added to 3 g of the cellulose raw material C. Pulverization was performed for 10 minutes using a precision dispersion/emulsification machine (M Technique Co., Ltd.; LIP-seal type laboratory equipment) as a mixer. A fine cellulose raw material Cf1 was thereby obtained (sample Cf1).

At the pulverizing step S3 using a mortar, pulverization was performed in a mortar for 30 minutes on 3 g of the cellulose raw material C. A fine cellulose raw material Cf2 was thereby obtained (sample Cf2).

In addition, at the pulverizing step S3 using a planetary ball mill, 40 mL of ultra-pure water was added to 3 g of the cellulose raw material C. A pulverization process involving a combination of 10 minutes of pulverization in a planetary ball mill apparatus (manufactured by Fritsch GmbH; PULVERISETTE 7 Classic Line) and 10 minutes of rest was performed six times in total. Suction filtration was performed thereafter. A fine cellulose raw material Cf3 was thereby obtained (sample Cf3).

At the enzymolysis step S4, 0.5 mL of sulfuric acid at a concentration of 72% was added to 50 mg each of the cellulose raw material C and the fine cellulose raw materials Cf1 to Cf3 (samples C and Cf1 to Cf3). Incubation was performed for 1 hour at 30°C. Respective cellulose raw material reaction solutions were thereby obtained. Each of the obtained cellulose raw material reaction solutions was placed in a high withstand pressure tube with 14 mL of ultra-pure water. Autoclave treatment was performed for 1 hour at 121 °C, Then, 5 g of calcium carbonate was added and a neutralization process was performed. Glucose was then collected. The method described in example 1 was used to quantitate the obtained glucose. The calculation results are shown in Fig. 7.

The samples shown in Fig. 7 are as follows: the cellulose raw material C after the washing step S2 is sample C; the fine cellulose raw material Cf1 obtained at the pulverizing step S3 using a mixer after the washing step S2 is sample Cf1; the fine cellulose raw material Cf2 obtained at the pulverizing step S3 using a mortar after the washing step S2 is sample Cf2; and the fine cellulose raw material Cf3 obtained at the pulverizing step S3 using a planetary ball mill after the washing step S2 is sample Cf3. Fig. 7 shows the glucose yields obtained by performing the enzymolysis process on each sample.

In the present example, as shown in Fig. 7, the glucose yields obtained after the enzymolysis process from 1 g of each of the samples C and Cf1 to Cf3 upon drying are: 1.09 mg of glucose from sample C; 15.07 mg of glucose from sample Cf1; 43.30 mg of glucose from sample Cf2; and 51.25 mg of glucose from sample Cf3. The values indicated in the Fig. 7 are the mean values of three samples, and the standard deviations thereof are indicated as error bars.

Based on the results, it is clear that the glucose production quantity increases as a result of the pulverizing step S3 being performed using a planetary ball mill. A reason for this is thought to be that, because the particle size of the fine cellulose raw material Cf3 after the pulverizing step S3 using the planetary ball mill is smaller than that when the mixer or the mortar is used, and the surface area increases, the portions in contact with the enzyme increases and enzymolysis is facilitated, thereby resulting in an increase in the production quantity of glucose.

<Example 5>

In the present example, an instance in which an alkaline hydrothermal treatment step is performed on the cellulose raw material C after the washing step S2 will be described. In the present example, in addition to the sterilizing step S I, the washing step S2, the pulverizing step S3, and the enzymolysis step S4 shown in Fig. 1, an alkaline hydrothermal treatment step using a sodium hydroxide solution was performed after the washing step S2. Glucose was then produced using the obtained cellulose raw material. Processes such as those below were performed at the respective steps.

First, at the sterilizing step S1, 200 g of bovine feces D was placed in a deep petri dish. Autoclave treatment was performed for 20 minutes at saturation water vapor pressure at 120°C. Sterilized bovine feces Ds was thereby obtained.

At the washing step S2, 50 g of the sterilized bovine feces Ds was filtered and washed using a gauze and 3 L of ultra-pure water (> 18.2 MΩcm$^{-1}$). Then, the sterilized bovine feces Ds was dried for 16 hours at 50°C. 150 mL of ultra-pure water was added to the obtained dried matter. A mixing and pulverizing process was performed in a mixer at 15000 rpm for 10 minutes. The obtained pulverized matter was then separated into 50 mL units and centrifuged for 10 minutes at 27000 G at 4°C. The obtained precipitate was then dried for 16 hours at 50°C, and the cellulose raw material C was thereby obtained from the sterilized bovine feces Ds.

At the alkaline hydrothermal treatment step, 90 mL of a sodium hydroxide solution at a concentration of 1% was added to 10 g of the cellulose raw material C. Autoclave treatment was performed for 5 minutes at saturation water vapor pressure at 120°C. Suction filtration was performed thereafter, and a post-alkaline hydrothermal treatment cellulose raw material was thereby obtained.

At the enzymolysis step S4 in the present example, 0.5 mL of sulfuric acid at a concentration of 72% was added to 50 mg of the post-alkaline hydrothermal treatment cellulose raw material. Incubation was performed for 1 hour at 30°C. A cellulose raw material reaction solution was thereby obtained. The obtained cellulose raw material reaction solution was placed in a high withstand pressure tube with 14 mL of ultra-pure water. Autoclave treatment was performed for 1 hour at 121°C. Then, 5 g of calcium carbonate was added and a neutralization treatment was performed. Glucose was then collected (sample A).

In addition, in the present example, to examine the effects of the alkaline hydrothermal treatment step and the pulverizing step S3 using the planetary ball mill, the post-alkaline hydrothermal treatment cellulose raw material obtained at the alkaline hydrothermal treatment step was pulverized at the pulverizing step S3 using the planetary ball mill described in example 4. The enzymolysis step S4 was then performed on the obtained fine cellulose raw material Cf4, and glucose was then collected (sample B).

Furthermore, to examine the effects of the alkaline hydrothermal treatment step, a sample A' in which glucose was collected after the enzymolysis process was performed on the cellulose raw material C obtained at the washing step S2, and a sample B' in which glucose was collected after the enzymolysis process was performed on the fine cellulose raw material Cf3 obtained by the pulverizing process using a planetary ball mill being performed on the cellulose raw material C in example 4 were prepared as samples for comparison.

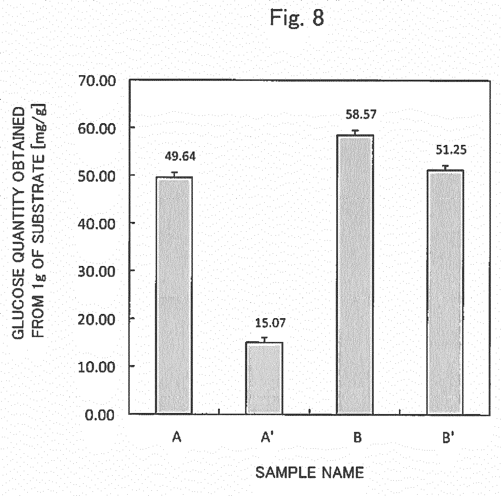

The glucose content in each sample calculated using the glucose quantitative method described in example 1 is shown in Fig. 8. In the present example, as shown in Fig. 8, from 1 g of each of the samples upon drying: 49.64 mg of glucose was obtained from sample A in which the post-alkaline hydrothermal treatment cellulose raw material obtained after the alkaline hydrothermal treatment step was used; 15.07 mg of glucose was obtained from sample A' in which the cellulose raw material C obtained after the washing step S2 was used; 58.57 mg of glucose was obtained from sample B in which the fine cellulose raw material Cf4 obtained after the pulverizing step S3 using a planetary ball mill was performed after the alkaline hydrothermal treatment step was used; and 51.25 mg of glucose was obtained from sample B' in which the fine cellulose raw material Cf3 obtained after the pulverizing step S3 using the planetary ball mill was performed after the washing step S2 was used. The values indicated in the Fig. 8 are the mean values of three samples, and the standard deviations thereof are indicated as error bars.

Based on the results, the results of the glucose yields of sample A and sample A' clearly indicate that the glucose yield can be significantly increased as a result of the alkaline hydrothermal treatment step being performed. A reason for this is thought to be that lignin, which inhibits decomposition of cellulose by the cellulase, is removed by the alkaline hydrothermal treatment. The cellulose becomes fibrous, and the distance between the substrate and the enzyme is shortened.

In addition, the results of the glucose yields of sample A, sample B, and sample B' clearly indicate that the glucose yield can be further increased by the alkaline hydrothermal treatment and the pulverizing step S3 using a planetary ball mill being combined. A reason for this is thought to be that, in addition to the effects of the alkaline hydrothermal treatment such as that described above, because the particle size of the substrate can be further reduced as a result of the pulverizing step S3 using a planetary ball mill, a synergetic effect of the increase in surface area of the substrate and the shortening of the distance between the substrate and the enzyme is achieved.

<Example 6>

In the present example, an instance in which the pulverizing step S3 is performed using a planetary ball mill and the enzymolysis step S4 is performed using two types of degradative enzymes will be described. In the present example, taking into consideration the outflow at the washing step S2 described in Fig. 1 of fine components and water-soluble components that potentially become the cellulose raw material C, glucose was produced by the steps excluding the washing step S2, that is, by the sterilizing step S1, the pulverizing step S3 using a planetary ball mill, and the enzymolysis step S4. Processes such as those below were performed at the respective steps.

First, the cellulose raw material C was obtained at the sterilizing step S1 using the method described in example 4.

At the pulverizing step S3, 20 mL of 50 mN sodium acetate buffer solution (pH - 4.0) was added to 1.33 g of the cellulose raw material C (water content of 83.5%) in a 45 mL zirconia container in which 18 zirconia balls having a diameter of 10 mm are enclosed. A pulverization process involving a combination of 10 minutes of pulverization in a planetary ball mill apparatus and 10 minutes of rest was performed six times in total. A 1 wt% ball mill-processed substrate solution was thereby obtained. Pulverization in the planetary ball mill apparatus was performed at two rotation speeds, 450 rpm and 600 rpm.

At the enzymolysis step S4, 200 $\mu$L of the 1 wt% ball mill-processed substrate solution and 50 $\mu$L of the cellulolytic enzyme solution were placed in a 1.5 mL microtube. 24-hour incubation was performed at 50°C. A sample was thereby prepared. Glucose quantitation was performed using the method described in example 1. As the cellulolytic enzyme solution, 0.5, 1, 5, 10, 50, or 100 mg/mL of enzyme / 50 mM sodium acetate buffer solution was used. The enzyme was Trichoderma viride (manufactured by Sigma-Aldrich Corporation; code: C9422-10KU) or Meiselase (manufactured by Meiji Co., Ltd). Regarding the cellulolytic enzyme concentration, because 50 $\mu$L of the enzyme solution is added to 250 mL of the reaction system (1 wt% ball mill-processed substrate solution + cellulolytic enzyme solution), the final concentration of the cellulolytic enzyme used is 0.1, 0.2, 1, 2, 10, or 20 mg/mL in the reaction system. Hereafter, the cellulolytic enzyme concentration is described as being 0.1, 0.2, 1, 2, 10, or 20 mg/mL.

Here, in the present example, to clarify the production quantities of glucose based on the differences in the enzyme that is used, a value obtained by subtracting the glucose quantity contained in an enzyme from an apparent glucose quantity is indicated as the actual glucose quantity obtained in the present example, with the glucose quantity collected from the sample obtained at above-described enzymolysis step S4 as the apparent glucose quantity. Table 1 indicates the apparent glucose quantity, the glucose quantity in the enzyme, and the actual glucose quantity obtained using Trichoderma viride. Table 2 indicates the apparent glucose quantity, the glucose quantity in the enzyme, and the actual glucose quantity obtained using Meiselase. The values indicated in table 1 and table 2 are mean values of three samples.

[Table 1]

| Rotation speed [rpm] | Enzyme concentration [mg/mL] | Glucose quantity [mg] | | |
|---|---|---|---|---|
| | | Apparent production quantity | Within enzyme | Actual production quantity |
| 450 | 0.1 | 41.0 | 3.6 | 37.4 |
| | 0.2 | 53.4 | 4.9 | 48.6 |
| | 1 | 78.8 | 19.5 | 9.3 |
| | 2 | 99.5 | 41.4 | 58.1 |
| | 10 | 250.1 | 184.5 | 65.5 |
| | 20 | 436.8 | 362.9 | 73.9 |
| 600 | 0.1 | 49.7 | 3.6 | 46.1 |
| | 0.2 | 57.9 | 4.9 | 53.1 |
| | 1 | 87.7 | 19.5 | 68.2 |
| | 2 | 100.9 | 41.4 | 59.6 |
| | 10 | 249.1 | 184.5 | 64.5 |
| | 20 | 494.6 | 362.9 | 131.7 |

[Table 2]

| Rotation speed [rpm] | Enzyme concentration [mg/mL] | Glucose quantity [mg] | | |
|---|---|---|---|---|
| | | Apparent production quantity | Within enzyme | Actual production quantity |
| 450 | 0.1 | 26.7 | 0 | 26.7 |
| | 0.2 | 32.0 | 0 | 32.0 |
| | 1 | 41.5 | 0 | 41.5 |
| | 2 | 47.9 | 0 | 47.9 |
| | 10 | 89.3 | 2.6 | 86.7 |
| | 20 | 138.4 | 8.7 | 129.8 |
| 600 | 0.1 | 38.6 | 0 | 38.6 |
| | 0.2 | 45.5 | 0 | 45.5 |
| | 1 | 63.7 | 0 | 63.7 |
| | 2 | 80.3 | 0 | 80.3 |
| | 10 | 106.2 | 2.6 | 103.6 |
| | 20 | 161.7 | 8.7 | 153.0 |

As indicated in table 1 and table 2, the glucose yield increases as the concentration of the cellulolytic enzyme increases. The sample in which Meiselase at a concentration of 20 mg¥mL was used as the cellulolytic enzyme and pulverization was performed at a rotation speed of 600 rpm had the highest glucose yield. 153.0 mg of glucose was obtained per 1 g of the substrate.

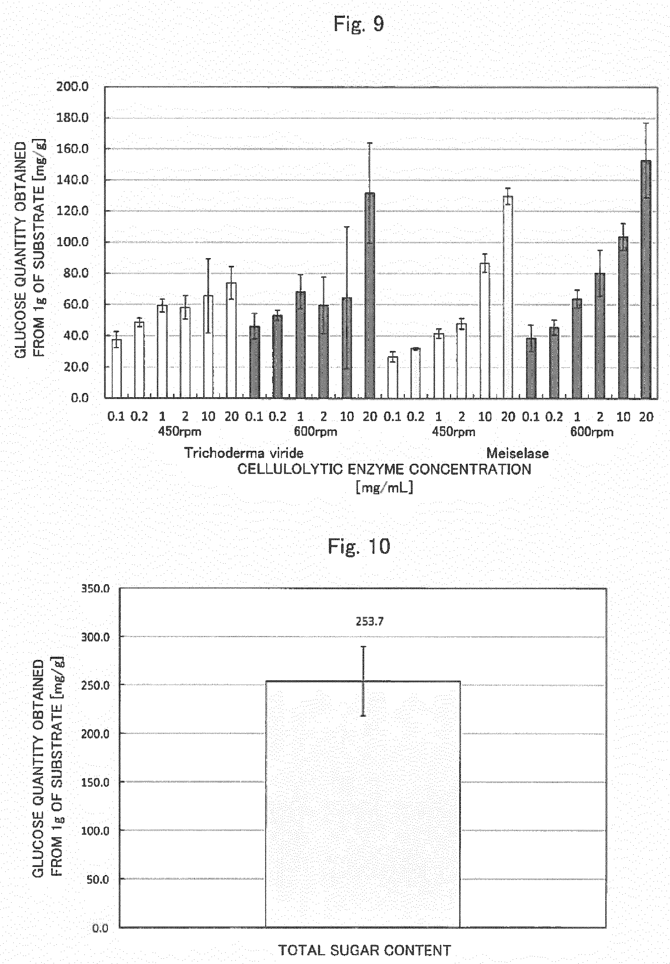

To compare the glucose quantities that are actually obtained based on the differences in the cellulolytic enzyme, the substantial glucose quantities in table 1 and table 2 are indicated in Fig. 9. The values in Fig. 9 are mean values of three samples, and the standard deviations thereof are indicated as error bars. In the present example, as shown in Fig. 9, it is clear that, with any of the cellulolytic enzymes, more glucose can be collected when pulverization is performed at a rotation speed of 600 rpm at the pulverizing step S3, compared to when pulverization is performed at a rotation speed of 450 rpm.

<Bovine feces total sugar content analysis>

Here, bovine feces total sugar content analysis was performed to clarify the total glucose quantity contained in the bovine feces D, by completely decomposing the bovine feces D to glucose using a common method. In the present analysis, decomposition was performed using dried sterilized bovine feces Ds that has undergone only the sterilizing step S1. A detailed bovine feces decomposing step will be described below.

50 mg of dried sterilized bovine feces Ds and 0.5 mL of sulfuric acid at a concentration of 72% was enclosed in a 2 mL microtube. Stirring was performed for 1 hour at 30°C in a vortex mixer. Next, the obtained sulfuric acid resolutive substrate of the sterilized bovine feces Ds and 14 mL of pure water were enclosed in a 20 mL heat-resistant, pressure-resistant screw-cap glass tube. Autoclave treatment was performed for 1 hour at 121 °C.

2 mL of the supernatant liquid after the autoclave treatment was placed in a 15 mL microtube. A neutralization process was performed by adding a suitable quantity of calcium carbonate until the pH level became neutral. Then, centrifugation was performed for 2 minutes at 15000 rpm using a high-speed centrifuge. A bovine feces decomposition solution was thereby obtained. Calculation of the glucose quantity was performed using the glucose reagent (LabAssay (registered trademark) Glucose, manufactured by WAKO Pure Chemical Industries, Ltd.) described in example 1. The obtained results are indicated in Fig. 10. The values indicated in the Fig. 10 are the mean values of three samples, and the standard deviations thereof are indicated as error bars.

The bovine feces total sugar content obtained from 1 g of the dried sterilized bovine feces Ds was revealed to be 253.7 mg, as shown in Fig. 10.

The glucose production method of the present invention is not limited to the above-described embodiment and examples. Various modifications are possible to an extent that the characteristics of the invention are not compromised.

EXPLANATIONS OF LETTERS OR NUMERALS

1 glucose production method
S1 sterilizing step
S2 washing step
S3 pulverizing step
S4 enzymolysis step
S5 glucose purifying step
X excrement
Xs sterilized excrement
C cellulose raw material
Cf fine cellulose raw material
D bovine feces
Ds sterilized bovine feces
H hay
Hf fine hay

**Claims**

1. A glucose production method **characterized in that**:

   glucose is produced from excrement from living matter.

2. The glucose production method according to claim 1, **characterized in that**:

   glucose is produced from cellulose contained in the excrement.

3. The glucose production method according to claim 2, **characterized in that**:

   a decomposing step of decomposing the cellulose contained in the excrement to glucose using a cellulolytic enzyme is performed.

**4.** The glucose production method according to claim 3, **characterized in that**:

a pulverizing step of pulverizing the cellulose contained in the excrement is performed before the decomposing step.

**5.** The glucose production method according to claim 4, **characterized in that**:

at the pulverizing step, the cellulose contained in the excrement is pulverized into fine particles having a diameter of 40 $\mu$m or less.

**6.** Glucose that **characterized by** being produced from excrement from living matter by a glucose production method according to any of claims 1 to 5.

Fig. 1

EXCREMENT

S1 ——— STERILIZING STEP

Xs

S2 ——— WASHING STEP

C

S3 ——— PULVERIZING STEP

Cf

S4 ——— ENZYMOLYSIS STEP

S5 ——— GLUCOSE PURIFYING
STEP

GLUCOSE

# Fig. 2

Ds

> 1mm

150μm

(a)

C

< 500μm

150μm

(b)

< 40μm

Cf

150μm

(c)

# Fig. 3

## Fig. 4

## Fig. 5

Fig. 6

Fig. 7

Fig. 8

## Fig. 9

## Fig. 10

<table>
<tr><td colspan="2" align="center">**INTERNATIONAL SEARCH REPORT**</td><td colspan="2">International application No.<br>PCT/JP2014/062328</td></tr>
</table>

**A. CLASSIFICATION OF SUBJECT MATTER**
*C12P19/14*(2006.01)i, *B09B3/00*(2006.01)i, *C02F11/02*(2006.01)i, *C13K1/02*
(2006.01)i

According to International Patent Classification (IPC) or to both national classification and IPC

**B. FIELDS SEARCHED**

Minimum documentation searched (classification system followed by classification symbols)
C12P19/14, B09B3/00, C02F11/02, C13K1/02

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)
JSTPlus/JMEDPlus/JST7580(JDreamIII), CAplus/MEDLINE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X | JP 2007-185117 A (Kyushu University),<br>26 July 2007 (26.07.2007),<br>claims 1 to 3; paragraphs [0014], [0024],<br>[0036] to [0042]<br>(Family: none) | 1-6 |
| X<br>Y | Champagne P, Bioethanol from Agricultural Waste<br>Residues, Enviromental Progress, Vol.27, No.1,<br>p.51-57(2008) | 1-4,6<br>5 |
| X<br>Y | Popiel et al., Ethanol production from maize<br>silage as lignocellulosic biomass in<br>anaerobically digested and wet-oxidized manure,<br>Bioresource Technology, Vol.99, p.5327-5334<br>(2008) | 1-4,6<br>5 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>10 July, 2014 (10.07.14) | Date of mailing of the international search report<br>22 July, 2014 (22.07.14) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/062328

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Yue et al., A Sustainable Pathway of Cellulose Ethanol Production Integrating Anaerobic Digestion With Biorefining, Biotechnology and Bioengineering, Vol.105, No.6, p.1031-1039 (2010) | 1-4,6<br>5 |
| X<br>Y | Maclellan et al., Anaerobic treatment of lignocellulosic material to co-produce methane and digested fiber for ethanol biorefining, Bioresource Technology, Vol.130, p.418-423 (2013.02) | 1-4,6<br>5 |
| X<br>Y | Yue et al., Development of a new bioethanol feedstock-Anaerobically digested fiber from confined dairy operations using different digestion configurations, Biomass and Bioenergy, Vol.35, p.1946-1953(2011) | 1-4,6<br>5 |
| Y | JP 2008-274247 A (National Institute of Advanced Industrial Science and Technology), 13 November 2008 (13.11.2008), claims; paragraph [0049]; examples & US 2010/0151527 A1 & EP 2133366 A1 & WO 2008/123419 A1 | 5 |
| Y | JP 2012-193353 A (National Institute of Advanced Industrial Science and Technology), 11 October 2012 (11.10.2012), claims; paragraphs [0032], [0041] to [0046]; examples (Family: none) | 5 |
| P,X | Ryo YOSHIMOTO et al., "Haikibutsukei Biomass kara no Glucose Sanseiho no Kento", The Cellulose Society of Japan Dai 20 Kai Nenji Taikai Koen Yoshishu, pages 132 to 133 (2013.07) | 1-6 |

Form PCT/ISA/210 (continuation of second sheet) (July 2009)

**EP 2 995 685 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011519550 A

- JP 2006149343 A